# EUROPEAN PATENT SPECIFICATION

(11) **EP 4 085 817 B1**
(45) Date of publication and mention of the grant of the patent: **04.06.2025**
(21) Application number: 22169465.6
(22) Date of filing: 22.04.2022
(51) Int. Cl.: A61B 1/00, A61B 1/05

(54) **ENDOSCOPE HEADS WITH LIGHT-PERMEABLE HOUSING AND METHOD OF MANUFACTURING ENDOSCOPE HEADS**
ENDOSKOPKÖPFE MIT LICHTDURCHLÄSSIGEM GEHÄUSE UND VERFAHREN ZUR HERSTELLUNG VON ENDOSKOPKÖPFEN
TÊTES D'ENDOSCOPE DOTÉ D'UN BOÎTIER PERMÉABLE À LA LUMIÈRE ET PROCÉDÉ DE FABRICATION DE TÊTES D'ENDOSCOPE

(30) Priority: 06.05.2021 US 202117313940
(43) Date of publication of application: 09.11.2022
(73) Proprietor: Karl Storz Endovision, Inc., Charlton, MA 01507 (US)
(72) Inventor: Birnkrant, Dashiell, MA, Sutton (US)
(74) Representative: Weidner Stern Jeschke

(56) References cited:
- US-A1- 2005 043 589
- US-A1- 2007 249 907
- US-B1- 6 293 910

## Description

The invention relates to endoscopes, endoscope heads, and manufacturing techniques for endoscope heads. Endoscope heads within the scope of the present invention have particular application in disposable endoscopes.

Observation instruments such as endoscopes are used in medical and other applications to allow a visual inspection of locations that are not readily accessible. For example, endoscopes are used in medical applications to provide a view of an area within a patient's body. An endoscope typically includes an elongated shaft of relatively small diameter extending from a handle to a distal end. An imaging or viewing means included with the endoscope allows a user to obtain a view from the distal end. In many modern endoscopes, the imaging or viewing means includes an electronic imaging device (also referred to herein as an image sensor-based camera) mounted in a distal head at the distal end of the endoscope. Distal head materials are typically limited to materials such as stainless steel, ceramic or polyether ether ketone (PEEK), as these materials are robust enough to withstand the rigors of cleaning and reprocessing, such as hydrogen peroxide gas-plasma sterilization, necessary in the medical industry. The electronic imaging device of such an endoscope, often referred to as a video endoscope or a COTT (chip on the tip) endoscope, collects image data and communicates that data through the shaft and handle ultimately to a processing system that converts the collected data into an image to be displayed on a suitable display device.

To provide the desired illumination for the image to be collected, light may be generated by a light source either contained within the handle or connected thereto and directed through suitable conduits in the shaft (such as optical fibers for example) to the endoscope distal head where the light can be directed as desired through suitable lenses. Alternatively to relying on light conducted through the shaft to the distal end of the endoscope, a suitable light source such as an LED lamp may be placed at the distal head of the endoscope together with the electronic imaging device to provide the required illumination.

In addition to the imaging or viewing arrangement and the illumination arrangement, some endoscopes and similar instruments include a working channel which usually extends from the instrument handle through the elongated shaft to the distal head. This working channel comprises a passageway through which fluids may be introduced into the area under observation during the endoscopic procedure, or through which a suction may be applied. The working channel may also be used to insert tools into the area under observation for performing certain functions in the course of the endoscopic procedure.

The distal head of an endoscope may be constructed as a housing that receives the various components, namely, the electronic imaging device and associated optics and electronics, illumination components, and a portion of the working channel passageway. Although it is necessary to provide room in the distal head housing for the imaging device, illumination components, and working channel, the distal head of the instrument ideally has a cross-sectional dimension that remains as small as possible in order to facilitate insertion into narrow cavities, minimizing the invasiveness of the desired observation and other procedures intended for the instrument. This is true in general, but especially for medical applications. This requirement for a small cross-sectional dimension at the distal head makes fabrication challenging. Not only does the small size make the distal head housing itself more difficult to fabricate, but the small size also complicates the assembly of the various components in the distal head housing.

US 2007/249907 A1 discloses an imaging assembly with a transparent distal cap positioned at the distal end of an endoscope, and an image sensor insert received partially within the transparent distal cap, wherein the distal cap and the image sensor insert are secured to a distal most ring of an articulating endoscopic shaft. Whereas the image sensor insert and the distal most ring are not transparent, only adhesive connections within the assembly for housing the imaging components can be cured by the application of curing energies, such as ultraviolet light, into the distal tip.

There remains a need for endoscope distal head structures that can accommodate all of the various endoscope head components desired for a given application and still be fabricated economically in a form that meets applicable size limitations. The desirability for more economical fabrication is particularly important for disposable endoscopes intended for only a single use.

The objective of the present invention is to improve the known state of the art.

The problem is solved by a distal head for an endoscope, the distal head including:
a distal head housing defining a distal end surface of the distal head and being formed from a light-permeable material that is permeable to light within a securing material curing spectrum;
a working channel tube receiving cavity defined within the distal head housing;
a working channel tube secured within the working channel tube receiving cavity with a first light-cured securing material cured in response to light within the securing material curing spectrum;
a camera receiving cavity defined within the distal head housing;
an image sensor-based camera assembly secured within the camera receiving cavity with a second light-cured securing material cured in response to light within the securing material curing spectrum;
an illumination light spread lens integrally formed in the distal head housing from the light-permeable material;
an illumination light conduit receiving cavity defined within the distal head housing; and
an illumination light conduit secured within the illumination light conduit receiving cavity with a third light-cured securing material cured in response to light within the securing material curing spectrum.

An endoscope distal head (which may be referred to herein simply as a "distal head") according to one aspect of the present invention includes a distal head housing defining a distal end surface of the distal head and being formed from a light-permeable material that is permeable to light at least within a securing material curing spectrum. The distal head housing defines a working channel tube receiving cavity, a camera receiving cavity, and an illumination light conduit receiving cavity. A working channel tube is secured within the working channel tube receiving cavity, while an image sensor-based camera assembly (which may be referred to as a "camera assembly") is secured within the camera receiving cavity and an illumination conduit is secured within the illumination light conduit receiving cavity. Each of these components, the working channel tube, the camera assembly, and the illumination light conduit are each secured in their respective receiving cavity with a light-cured securing material that has been cured by exposure to light within the securing material curing spectrum.

The use of the light-permeable material to form the distal head housing allows all of the various components secured to the housing, namely, the working channel tube, camera assembly, and illumination light conduit to be readily secured in place with the light-cured securing material. As described further below, each of the components may be placed in the desired operating position within the respective receiving cavity together with a sufficient amount of light-curable securing material, that is, material that may be cured by exposure to light within the securing material curing spectrum. The light-curable securing material may then be cured, that is, converted to the light-cured securing material by directing light within the securing material curing spectrum through the distal head housing material. Thus, a distal head according to the first aspect of the invention provides additional options for fabrication of the device and these options may be particularly helpful in situations where the transverse dimension of the distal head must be small.

According to another aspect of the invention, an endoscope includes an elongated shaft having a distal end and a proximal end with an endoscope handle connected at the proximal end of the endoscope shaft and a distal head according to the first aspect of the invention connected at the distal end of the endoscope shaft. A working channel tube, camera assembly, and illumination light conduit are each secured in their respective receiving cavity within the distal head housing with light-cured securing material as described above in reference to the first aspect of the invention. The working channel tube extends through the shaft to a working channel access structure of the endoscope handle, while the illumination light conduit extends through the shaft to the endoscope handle where it is operably coupled to an illumination light source usually associated with the endoscope handle. The camera assembly is connected to a power and signal conduit arrangement that extends through the endoscope shaft to an operative connection within the endoscope handle.

Furthermore, the problem is solved by methods of manufacturing a distal head for an endoscope according to claims 11 to 13. Methods according to this aspect of the invention include securing a distal head housing formed from a light-permeable material as described above in an assembly position. These methods then include placing a first endoscope head component and two more endoscope head components such as the above-described camera assembly, working channel and illumination light conduit in an operating position within the respective component cavities within the distal head housing together with respective light-curable securing material interposed between a respective surface of the respective component cavity and an outer surface of the respective endoscope head component. While the first endoscope head component is maintained in the operating position within the first component cavity with the light-curable securing material, methods according to this aspect of the invention further include directing light within the securing material curing spectrum through the light-permeable material of the distal head housing to the light-curable securing material. The light so directed cures the light-curable securing material to form the light-cured material and thereby secure the first endoscope head component in the operating position within the first component cavity.

Methods according to this aspect of the invention encompass numerous variations. For example, some methods may include placing multiple different endoscope head components each within a respective component cavity formed in the distal head housing and each with the light-curable securing material interposed between the outer surface of the component and surface of the respective cavity. With multiple endoscope components thus positioned, the light within the securing material curing spectrum may be directed through the light-permeable material of the distal head housing to cure the securing material and thus secure the different components in place simultaneously. It is also possible within the scope of this third aspect of the invention to place a given endoscope head component and direct the appropriate curing light to secure the component in place one component at a time. Still further variations may place and secure a single component in place and place and secure multiple components in place either before or after securing the single component in place.

In implementations of an endoscope distal head according to any of the foregoing aspects of the invention, the light-permeable material from which the distal head housing is formed may be a material that is also permeable to light within a spectrum that includes an operating light spectrum for the camera assembly and the illumination light conduit. In these implementations the distal head may further include an illumination light spread lens located in the distal head housing with an inner face thereof facing and operatively aligned with an illumination light output of the illumination light conduit. Such an illumination light spread lens may comprise a plano-concave lens having a concave side comprising the inner face of the lens. The operating light spectrum for the camera assembly and the illumination light conduit may comprise the visible light spectrum for example.

Implementations of an endoscope distal head according to any of the forgoing aspects of the invention may use a plastic optical fiber as the illumination light conduit. Regardless of the specific form of the illumination light conduit, multiple such conduits may be desirable. In these cases, the distal head housing would include a respective illumination light conduit receiving cavity for each illumination light conduit, and each such conduit may be secured using the light-curable securing material.

The light-curable securing material employed according the any of the forgoing aspects of the invention may comprise a UV-curable epoxy which may be exposed to UV light to form the light-cured securing material. In this case the light-permeable material from which the distal head housing is formed is permeable to the UV spectrum or at least the applicable UV spectrum for the selected epoxy. In implementations where the distal head housing includes integral lenses formed from the light-permeable housing material, using a UV-curable material as the light-curable securing material would require that the light-permeable housing material would be permeable to both the operating light spectrum for given endoscope component and also the UV spectrum or applicable portion thereof.

These and other advantages and features of the invention will be apparent from the following description of representative embodiments, considered along with the accompanying drawings. The invention is further explained by the following exemplary description of particular embodiments. The figures show:
- FIG. 1: a view in perspective of a flexible shaft, video endoscope including a distal head according to an embodiment of the present invention,
- FIG. 2: a front right perspective view of the distal head of the endoscope shown in FIG. 1,
- FIG. 3: a perspective view similar to FIG. 2, but with the distal head shown in longitudinal section along line 3-3 in FIG. 2 to expose portions of the inner structure of the distal head,
- FIG. 4: a longitudinal section view along line 3-3 in FIG. 2 showing just the distal head housing,
- FIG. 5: a transverse section view of the distal head housing along line 5-5 in FIG. 4,
- FIG. 6: a longitudinal section view similar to FIG. 4 but with two endoscope head components each in their respective operative position within their respective receiving cavity prior to placement of the light-curable securing material,
- FIG. 7: a longitudinal section view along line 7-7 in FIG. 2 showing only the distal head housing with a plastic optical fiber in its operating position within its receiving cavity prior to placement of the light-curable securing material, and
- FIG. 8: a side view of a portion of the distal head shown in FIGS. 1-7 showing an illumination cone from one of the illumination light spread lenses and a field of view for the camera included in the distal head.

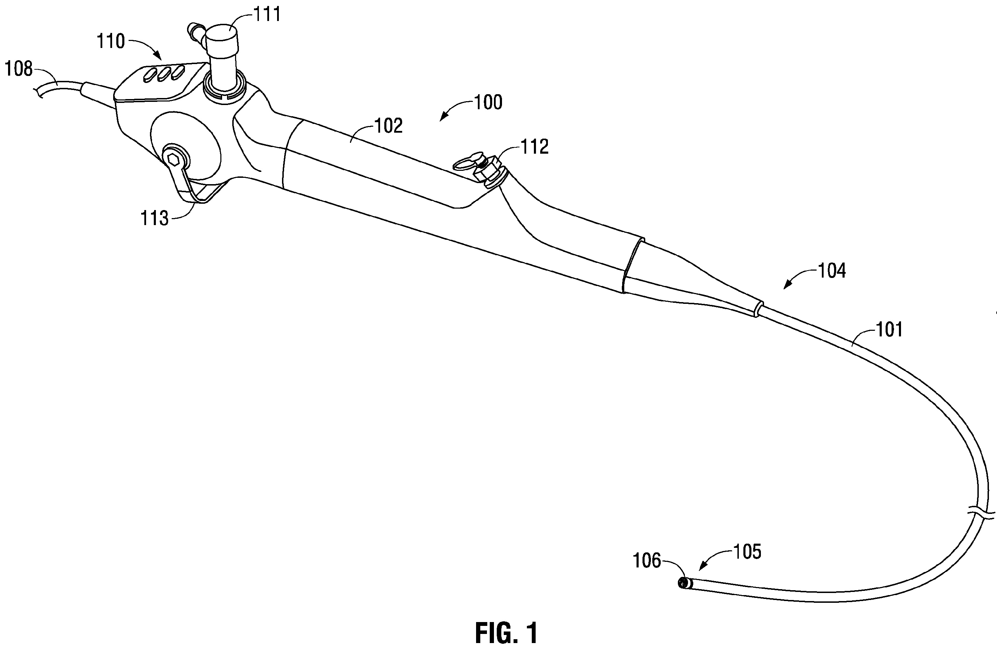

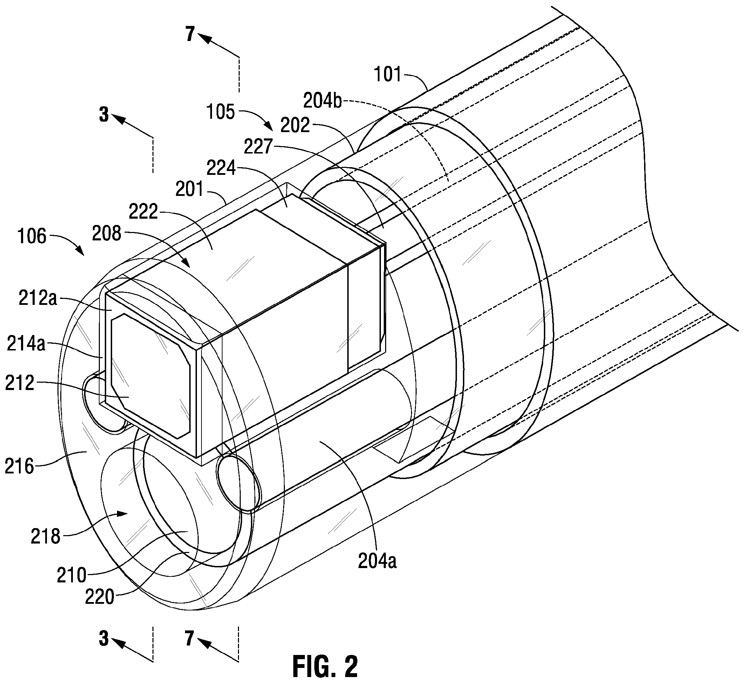

Referring to FIG. 1, an endoscope 100 according to one aspect of the present invention includes an elongated shaft 101 and a handle 102. Shaft 101 extends from a proximal end shown generally at reference numeral 104 connected to handle 102 to a distal end generally indicated at reference numeral 105. A distal head 106 is mounted at the shaft distal end 105 and includes a light-permeable housing in accordance with the present invention as will be described further below in connection with the larger-scale views of FIGS. 2-8.

Endoscope 100 receives electrical operating power through a cable 108. This power may be used to operate electronic components associated with distal head 106. Also, data signals from a camera assembly in distal head 106 may be communicated through appropriate pathways within shaft 101 and handle 102 to cable 108. In particular, a cable including a suitable number of electrical wires may extend from handle 102 through shaft 101 to the camera assembly contained within distal head 106. Data signals from the camera assembly may be communicated through cable 108 to processing equipment (not shown) that processes the image data and drives one or more video monitors to display the images collected at distal head 106.

Those familiar with endoscopic systems will appreciate that endoscope 100 includes a number of features such as controls 110 for controlling the operation of the endoscope and ports for introducing fluids or applying a suction to a working channel included in the endoscope. This particular endoscope shown is a flexible shaft endoscope and also has controls 113 for directing the end of the shaft. Example endoscope 100 includes ports 111 and 112 which are each in communication with a working channel extending from handle 102, through shaft 101, to distal head 106. Port 111 may comprise a coupling to allow a suction to be applied to the working channel or to allow fluids to be applied to the working channel. Port 112 may include an access opening through which an instrument may be inserted into the working channel. This working channel may be formed by a working channel tube which is not shown in FIG. 1 but will be described below in connection with the larger-scale figures. Endoscope handle 102 may also house an illumination light source or a connection to such a light source that directs illumination light through one or more illumination light conduits extending through shaft 101 to distal head 106. These illumination light conduits are not shown in FIG. 1 but will be shown and described below in connection particularly with FIGS. 2-5. Traditionally, illumination is provided to an object space by optical fibers acting as conduits. More recently, distally placed LEDs have been used. However, both conventional glass optical fibers and LEDs have drawbacks for modern designs, particularly in single-use endoscopic systems. Conventional fibers are expensive and the means to couple internal fibers with an external light source can be complicated. Distally placed LEDs are relatively large and limit the minimum size possible at the end face of the distal tip, which is of paramount concern. Additional features and the general operation and control of endoscope 100, as known in the art, will not be described further herein in order to avoid obscuring the present invention in unnecessary detail.

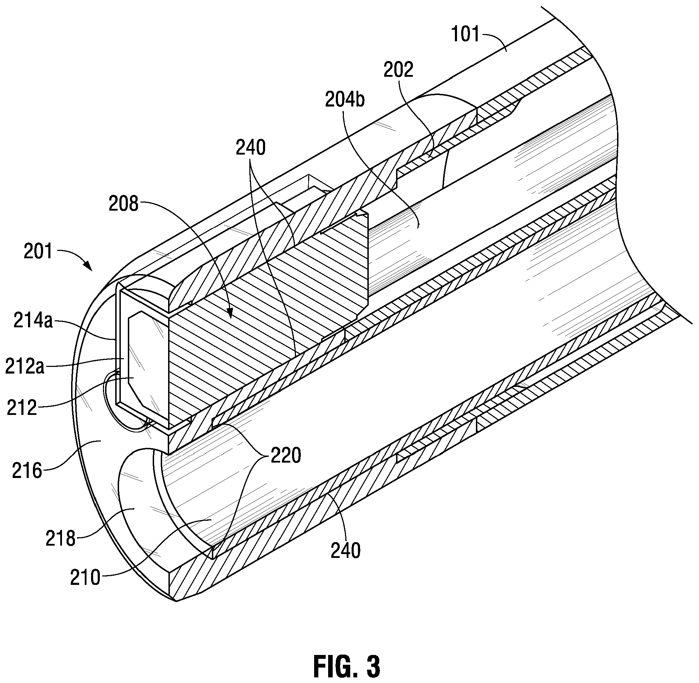

FIG. 2 shows distal head 106 connected to the distal end 105 of in the scope shaft 101. Distal head 106 includes a distal head housing 201 that is formed from a light-permeable material. FIG. 2 shows that the light-permeable material in this example is optically transparent, that is, permeable to light within the visible light spectrum. The transparent nature of the material from which distal head housing 201 is formed allows various internal components of distal head 106 to be visible in FIG. 2. In particular, FIG. 2 shows a connecting collar 202 that connects distal head housing 201 to shaft 101. A first illumination light conduit 204a is also visible in FIG. 2, with a second illumination light conduit 204b visible in the section view of FIG. 3. Each of these illumination light conduits in this example may comprise a plastic optical fiber (POF). Plastic optical fibers offer the benefit of being inexpensive while not taking up as much valuable distal face space as LEDs. However, POFs suffer from some drawbacks that have heretofore limited their efficacy in endoscopic systems. Namely, POFs are generally made of materials that have biocompatibility issues, and therefore should not be exposed to the human body during an endoscopic procedure. Further, while POFs transmit visible light very efficiently due to a large core diameter of usually greater than 0.25mm, they generally have a low numerical aperture (NA), resulting in a cone of illuminating light that does not generally encompass the large field of view desired for endoscopic procedures. The present invention overcomes these drawbacks such that POFs may be efficiently used as illumination conduits.

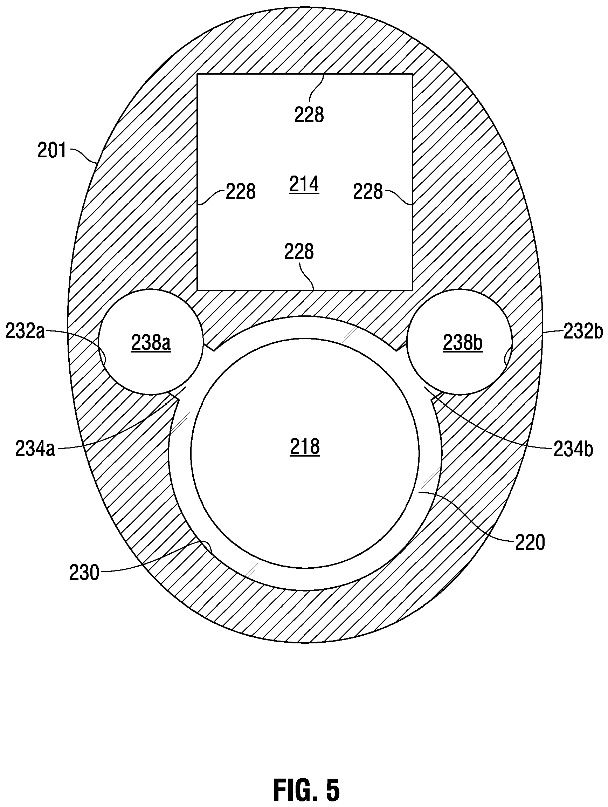

Both FIGS. 2 and 3 show a camera assembly 208 and a working channel tube 210. In this example embodiment, camera assembly 208 includes a cover lens 212 that is slightly larger in the transverse dimension than the remainder of the camera assembly 208 and is adapted to be received in a camera opening 214 formed in a distal surface 216 of distal head housing 201. An edge 214a of camera opening 214 is shown in FIGS. 2 and 3, while the camera opening 214 itself is shown particularly in Fig. 5. This example cover lens 212 includes opaque peripheral shielding 212a. Working channel tube 210 is received in distal head housing 201 so that it aligns with a working channel opening 218 formed in distal surface 216. As shown best in FIG. 3, the end of working channel tube 210 in this embodiment abuts a stop surface 220 formed in distal head housing 201.

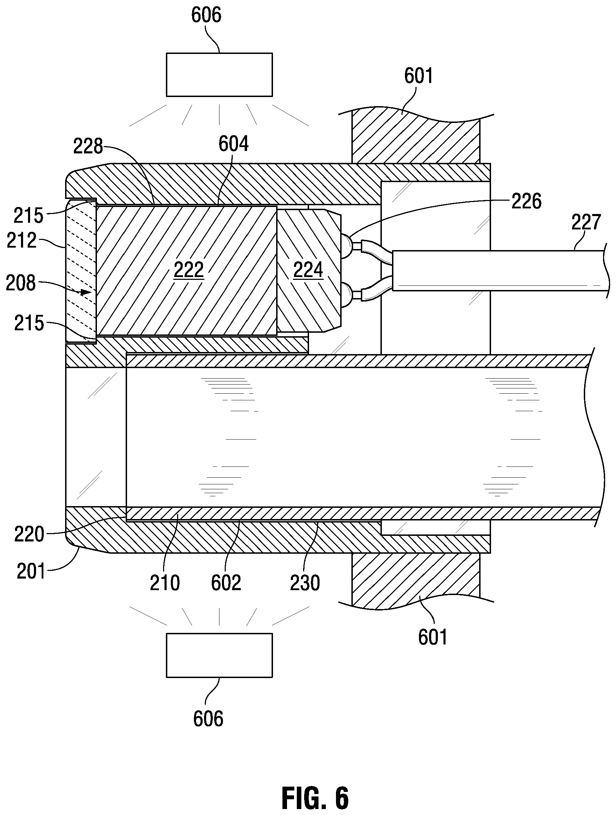

As is common in camera assemblies for endoscopes, camera assembly 208, in addition to cover lens 212, includes camera body 222 and an electronic image sensor package 224 both shown in FIG. 2. Camera body 222 may house a series of lenses that focus an image on an image sensor array within image sensor package 224. Camera assembly 208 further includes on its end opposite to cover lens 212 a number of electrical contacts 226 that connect to leads from a cable 227 (contacts 226 and cable 227 being visible in FIG. 6) that may extend through shaft 101 to the handle 102 shown in FIG. **1****. It** should be appreciated that camera assembly 208 is provided as an example, and that substantially any image sensor-based camera assembly may be used in an endoscope distal head according to the present invention.

Each of the distal head components, the camera assembly 208, each illumination light conduit 204a and 204b, and working channel tube 210 is received in a respective component receiving cavity formed within distal head housing 201. The various component receiving cavities are shown in several of the views but may perhaps most clearly be seen in the transverse section view of FIG. 5 that shows the distal head housing 201 prior to installation of the various components. Referring to FIG. 5, camera assembly receiving cavity in this example comprises the volume bounded by cavity surfaces 228 and has a roughly square transverse shape to match the transverse shape of camera body 222 and image sensor package 224. Camera opening 214 is visible in the direction of view for this section. A working channel tube cavity is bounded by generally cylindrical cavity surface 230 with the working channel stop surface 220 and working channel opening 218 both visible in the view of FIG. 5. A first illumination light conduit receiving cavity includes predominantly cylindrical cavity surface 232a while a second illumination light conduit receiving cavity includes predominantly cylindrical cavity surface 232b. Also as best shown in FIG. 5 but also apparent from some of the other views, the two illumination light conduit receiving cavities formed by surfaces 232a and 232b, respectively, and the working channel tube receiving cavity formed by surface 230 are connected by channels 234a and 234b, respectively, which in this example run along the entire length of each illumination light conduit receiving cavity.

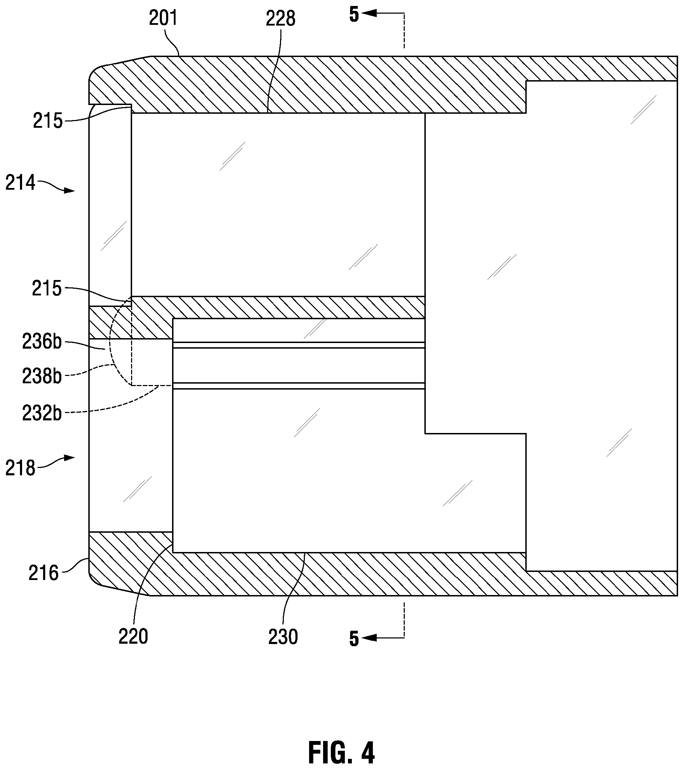

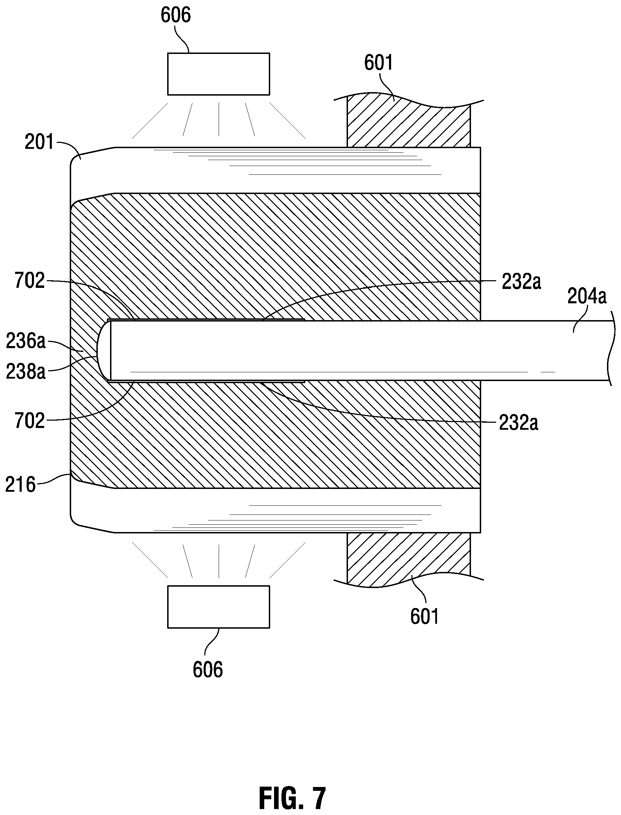

The example distal head housing 201 shown in the drawings includes illumination light spread lenses integrally formed with the distal head housing 201 from the light-permeable material. A spread lens 236a associated with illumination light conduit 204a is best shown in FIG. 7 while a spread lens 236b is best shown in the section view of FIG. 4 and is associated with illumination light conduit 204b shown in FIG. 3. This integral formation of lenses 236a and 236b is made possible employing material for distal head housing 201 that is permeable to the illumination light provided through the illumination light conduits 204a and 204b. Illumination light spread lenses 236a and 236b in this case are each a plano-concave lens having a concave inner surface facing the output end of the respective illumination light conduit, and a planar outer surface comprising a portion of distal surface 216. The transverse section view of FIG. 5 shows the concave surface 238a of lens 236a and the concave surface 238b of lens 236b, both in position to face the output end of the respective illumination light conduit that will be received in the respective receiving cavity (formed by surfaces 232a and 232b, respectively).

Each of the endoscope components, camera assembly 208, working channel tube 210, and illumination light conduits 204a and 204b are preferably secured in position within their respective receiving cavity. In accordance with the present invention, at least one such component, and preferably more than one, or all such components are secured in place with light-cured securing material as described above in the summary section. The section view of FIG. 3 shows light-cured securing material 240 as a thin layer interposed between the outer surface of camera assembly 208 and surface 228 forming the camera assembly receiving cavity. The light-cured securing material 240 is also visible in FIG. 3 as a thin layer interposed between the outer surface of working channel tube 210 and the surface 230 forming the working channel tube receiving cavity. In implementations according to the present invention, the light-cured securing material may be included along the entire length of the given receiving cavity surface and may be included continuously around the entire periphery of the given component, camera assembly 208, working channel tube 210, or illumination light conduit 204a or 204b. Alternatively, the securing material may be present along only a portion of the receiving cavity surface and along only a portion of the periphery of the given component. Light-cured securing material 240 need only be present along a sufficient portion of the periphery of the given component to secure the component in the desired operating position within the given receiving cavity so as to resist forces which may be encountered tending to move the component from the desired operating position. Such forces might be encountered either while manufacturing the remainder of the endoscope or using the endoscope.

The light-cured securing material may comprise a glue, adhesive, or other bonding material that bonds the respective component (camera assembly 208, working channel tube 210, or illumination light conduit 204a or 204b) to the surface forming the respective receiving cavity. The invention is not limited to any particular chemical or physical mechanism for providing the desired securing bond, however, to take advantage of the light-permeable nature of the material from which distal housing 201 is formed, the light-cured securing material according to the present invention is formed from a light-curable material that is cured when subjected to light within a spectrum to which the distal housing material is permeable. This use of light-curable securing material simplifies and reduces the time required to manufacture distal head 106, including enabling the ability for an assembler to visually inspect the assembly as it proceeds within the distal head, improving manufacturing precision thereby, as will be described below in connection with FIGS. 6 and 7.

Referring first to FIG. 6, a method within the scope of the invention for manufacturing distal head 106 includes first securing the distal head housing 201 in a desired assembly position. This securing in an assembly position may be accomplished with a suitable chuck or fixture shown at 601 in FIG. 6. With the distal head housing 201 so secured, the method includes placing at least one endoscope component in an operating position in its respective component receiving cavity. FIG. 6 shows both the endoscope component comprising the working channel tube 210 and endoscope component comprising the camera assembly 208 having been placed in the respective operating position. In this particular example, distal head housing 201 is configured so that working channel tube 210 may be inserted into its receiving cavity (formed by surface 230) from the right in the orientation of the figure and inserted until it abuts the stop surface 220. This example distal head housing 201 is also configured so that camera assembly 208 may be inserted from the left in the orientation of the figure until lens cover 212 abuts the camera cover stop surface 215. In the operating position of each component (camera assembly 208 and working channel tube 210) there remains a gap between the outer surface of the component and the inner surface of the respective cavity. This gap is apparent for working channel tube 210 and working channel tube receiving cavity surface 230 at 602 in FIG. 6, and is apparent for camera assembly 208 and camera assembly receiving cavity surface 228 at 604 in FIG. 6. The manufacturing method according to the present invention includes placing the respective component in its operating position with the light-curable securing material interposed between the outer surface of the component and surface of the respective cavity, that is in the gaps such as gaps 602 and 604 in FIG. 6. This placement of the light curable securing material may be accomplished by injecting the light-curable securing material into the respective gap once the component is in the operating position or by coating at least some of the cavity surface and/or portions of the exterior surface of the corresponding component with the light-curable securing material and then placing the component such as working channel tube 210 and camera assembly 208 in the respective operating position shown in FIG. 6.

Regardless of how the component or components are placed in their operating position with the light-curable securing material interposed between the component the respective receiving cavity surface, the method further includes directing light within the securing material curing spectrum through the light-permeable material of distal head housing 201 to the light-curable securing material to cure the material (form the light-cured material) to secure the given component in the desired operating position. FIG. 6 shows the curing light generated from light sources 606 placed around the transverse periphery of distal head housing 201. It will be appreciated particularly from FIG. 6 that the areas in which the light-curable securing material may be located, specifically the gaps 602 and 604 are not readily accessible to light from the longitudinal ends of distal head housing 201. Thus it would not be possible, or it would at least be difficult, to apply the curing light to the material at least in some places without directing the light through the material of housing 201. That is, it would not be possible or at least more difficult to secure the components in place with a light-curable securing material if the housing 201 was not formed from an appropriately light-permeable material. Directing the curing light from around the periphery of distal head housing 201 as shown in FIG. 6 ensures that the entire volume of the distal head housing is exposed to curing light to cure the light-curable securing material and secure the components in place. It should also be noted that the position of chuck or fixture 601 is such that it does not interfere with directing the curing light to the light-curable securing material within distal head housing 201.

The section view of FIG. 7 shows distal head housing 201 secured in position with chuck or fixture 601 and with first illumination light conduit 204a received in its operating position in the first illumination light conduit receiving cavity defined by surface 232a. In this example embodiment, distal head housing 201 is configured so that illumination light conduit 204a is inserted into the receiving cavity from the right in the orientation of the figure and slid into the receiving cavity until it abuts the end surface of the cavity. In this case this the end surface comprises the outer edges of concave surface 238a forming plano-concave lens 236a. It is apparent from the figure that a small gap 702 is left between the outer surface of illumination light conduit 204a and cavity surface 232a. The light curable securing material is placed in the gap 702 so as to be interposed between the outer surface of illumination light conduit 204a and cavity surface 232a. With illumination light conduit 204a so positioned with light-curable securing material interposed in at least part of gap 702, the manufacturing method includes directing curing light through the light-permeable housing material to the light-curable securing material to cure the material and secure illumination light conduit 204a in its operating position.

Methods according to the present invention are not limited to any particular technique for placing the conduit in the operating position with the light-curable securing material interposed between the required surfaces. The light-curable securing material may be injected into a gap such as gaps 602 and 604 in FIG. 6 or gap 702 in FIG. 7 after placement of the respective component (working channel tube 210, camera assembly 208, and illumination light conduit 204a, respectively) or positioned in the gap in any other suitable fashion. Other implementations may inject the light-curable securing material into the gap such as 602, 604 and 702 while the component is being inserted to the operating position. In some cases, it may be desirable to at least partially coat a surface of a receiving cavity and/or the outer surface of the corresponding component with the light-curable securing material prior to inserting the component into the receiving cavity. In these cases, the insertion of the component may serve to spread the light-curable securing material so as to better fill the gap between the component and cavity surface. Some implementations of method according to the invention may include both precoating a receiving cavity surface (and/or component outer surface) with the light-curable securing material and then injecting more of the light-curable securing material either after the component is placed in the operating position or while the component is being placed in the operating position. Regardless of how the light-curable securing material is placed in the gaps such as gaps 602, 604, and 702, as noted above in connection with FIG. 3, it may not be necessary to completely fill the given gap with the light-curable securing material. The various components should be bonded in place sufficiently to withstand the stresses encountered in further manufacturing or in use, but this may require only a small portion of a given gap be filled with the light-curable securing material.

In the example distal head housing 201 shown in the figures, the method may include placing each of the components in their respective operating position in their receiving cavity together with the positioned light-curable securing material and then curing that material to secure each of those components in the desired operating position simultaneously. Alternatively, each component may be inserted to the desired operating position and the light-curable securing material placed according to the invention and then cured to secure only that component in its desired operating position. The process may then be repeated for each additional component to be secured in its respective operating position. In other implementations it may be desirable to place a subset comprising two or more of components in their desired operating position together with the light-curable securing material and then that light-curable securing material cured to secure those components. One or more additional components may then be placed and secured with the light-curable securing material. For example, particularly with distal head housing 201 shown in the present figures, in which the receiving cavities for the working channel tube and illumination light conduits are connected (via channels 234a and 234b in FIG. 5), it may be desirable to place working channel tube 210 and illumination light conduits 204a and 204b and then secure them in place simultaneously by curing the light-curable securing material. This simultaneous securing of the working channel tube 210 and illumination light conduits 204a and 204b may be performed before or after camera assembly 208 is secured in place in accordance with the present invention.

The present invention may employ any light-curable securing material suitable for the given application. The primary requirement for the light-curable securing material is that it must be curable with light in a spectrum to which the distal head housing material is permeable. UV-curable epoxy may be used as the light-curable securing material where the distal head housing is formed from optically transparent polymers, such as acrylic. As discussed throughout the disclosure, these materials offer the distinct advantage over traditional endoscopic distal head materials in that they are transparent. Additionally, these materials are particularly useful when used in disposable endoscopic systems.

Distal head housings made from transparent polymers can be produced with a casting or mold injection process, which is not possible with some conventional materials. Also, the inventive distal heads described herein may be produced by 3D printing or other appropriate techniques. The ability to use these manufacturing techniques enables the integration of illumination spread lenses, discussed above, directly into the distal head, obviating, thereby the difficult process of inserting spread lenses into a machined piece, and enabling the inventive use of POFs as illumination conduits rather than much more expensive conventional optical fibers.

As discussed above, the NA of POF is generally much smaller than that of a conventional fiber bundle, resulting in a much smaller illumination cone. For example, a POF with a typical NA of 0.6 might have an illumination cone of only about 74 degrees, far below the typical endoscopic field of view of from 90-140 degrees. However, the present invention enables, as discussed above, the integration of spread lenses into the distal tip material itself, along with the illumination light conduit receiving cavity. Thus, an integrated spread lens can modify the effective NA of our example POF to 0.95, corresponding to an illumination cone of 145 degrees, making, thereby, the use of POFs practical for illumination conduits. As disposable scopes are single use in nature, they are not need to be required to survive the rigors of repeated chemical cleaning and/or autoclaving, and therefore need not be made from more robust materials such as stainless steel or PEEK. The present invention therefore enables the use of less expensive materials, more conducive to single-use systems, as well as overcomes problems associated with use of non-biocompatible materials, such as POFs.

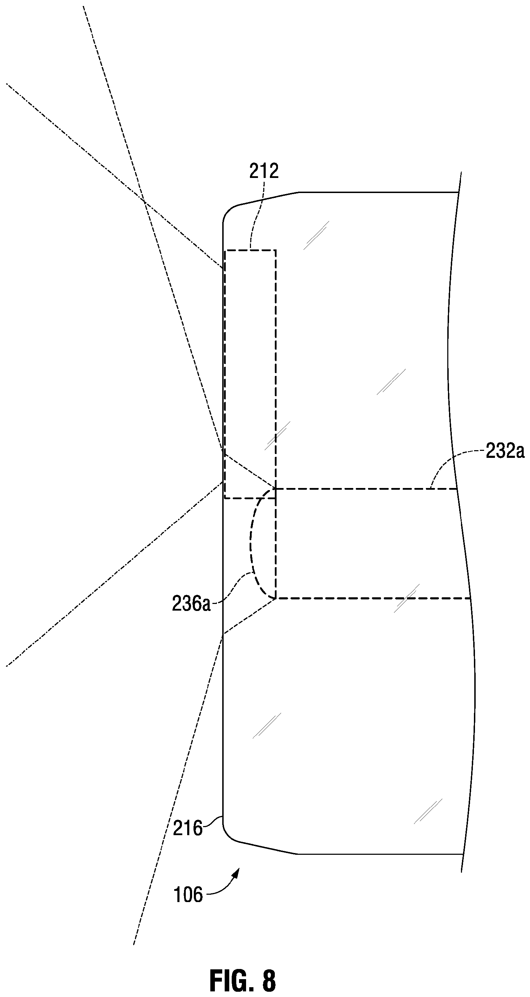

The example of FIG. 8 shows a portion of a distal head 106 with an illumination cone represented in dashed lines from the spread lens 236a associated with illumination light conduit receiving surface 232a. In this illustration, it is assumed that a POF is received in the illumination light conduit receiving surface 232a with an end of the POF facing the concave side 238a of lens 236a. FIG. 8 also shows dot-dash lines representing the field of view of the camera through camera lens cover 212. In this example of FIG. 8, the camera field of view is shown at approximately 140 degrees while the illumination cone produced by the combination of POF and spread lens 236a is shown as approximately 145 degrees. Thus, the field of illumination covers the camera field of view after only a small distance from the distal surface 216.

As used herein, whether in the above description or the following claims, the terms "comprising," "including," "carrying," "having," "containing," "involving," and the like are to be understood to be open-ended, that is, to mean including but not limited to. Also, it should be understood that the terms "about," "substantially," and like terms used herein when referring to a dimension or characteristic of a component indicate that the described dimension/characteristic is not a strict boundary or parameter and does not exclude variations therefrom that are functionally similar. At a minimum, such references that include a numerical parameter would include variations that, using mathematical and industrial principles accepted in the art (e.g., rounding, measurement or other systematic errors, manufacturing tolerances, etc.), would not vary the least significant digit.

Any use of ordinal terms such as "first," "second," "third," etc., in the following claims to modify a claim element does not by itself connote any priority, precedence, or order of one claim element over another, or the temporal order in which acts of a method are performed. Rather, unless specifically stated otherwise, such ordinal terms are used merely as labels to distinguish one claim element having a certain name from another element having a same name (but for use of the ordinal term).

The term "each" may be used in the following claims for convenience in describing characteristics or features of multiple elements, and any such use of the term "each" is in the inclusive sense unless specifically stated otherwise. For example, if a claim defines two or more elements as "each" having a characteristic or feature, the use of the term "each" is not intended to exclude from the claim scope a situation having a third one of the elements which does not have the defined characteristic or feature.

The above-described preferred embodiments are intended to illustrate the principles of the invention, but not to limit the scope of the invention. Various other embodiments and modifications to these preferred embodiments may be made by those skilled in the art without departing from the scope of the present invention. For example, in some instances, one or more features disclosed in connection with one embodiment can be used alone or in combination with one or more features of one or more other embodiments. More generally, the various features described herein may be used in any working combination.

### Reference numerals

- 100: endoscope
- 101: shaft
- 102: handle
- 104: proximal end of shaft
- 105: distal end of shaft
- 106: distal head
- 108: cable
- 110: controls
- 111: port
- 112: port
- 113: controls
- 201: distal head housing
- 202: connecting collar
- 204a: first illumination light conduit
- 204b: second illumination light conduit
- 208: camera assembly
- 210: working channel tube
- 212: cover lens
- 212a: peripheral shielding
- 214: camera opening
- 214a: edge
- 215: stop surface
- 216: distal surface
- 218: working channel opening
- 220: stop surface
- 222: camera body
- 224: image sensor package
- 226: electrical contacts
- 227: cable
- 228: cavity surface
- 230: cavity surface
- 232a: cavity surface
- 232b: cavity surface
- 234a: channel
- 234b: channel
- 236a: spread lens
- 236b: spread lens
- 238a: concave surface
- 238b: concave surface
- 240: light-cured securing material
- 601: chuck or fixture
- 602: gap
- 604: gap
- 606: light source
- 702: gap

## Claims

1. A distal head (106) for an endoscope (100), the distal head (106) including:
a distal head housing (201) defining a distal end surface of the distal head (106) and being formed from a light-permeable material that is permeable to light within a securing material curing spectrum;
a working channel tube receiving cavity defined within the distal head housing (201);
a camera receiving cavity defined within the distal head housing (201);
an illumination light spread lens (236a, 236b) integrally formed in the distal head housing (201) from the light-permeable material;
an illumination light conduit receiving cavity defined within the distal head housing (201); **characterized by**
a working channel tube (210) secured within the working channel tube receiving cavity with a first light-cured securing material (240) cured in response to light within the securing material curing spectrum;
an image sensor-based camera assembly (224) secured within the camera receiving cavity with a second light-cured securing material (240) cured in response to light within the securing material curing spectrum; and
an illumination light conduit (204a, 204b) secured within the illumination light conduit receiving cavity with a third light-cured securing material (240) cured in response to light within the securing material curing spectrum.

2. The distal head (106) of claim 1 wherein:
the light-permeable material is also permeable to light within a spectrum that includes an operating light spectrum for the image sensor-based camera assembly (224) and the illumination light conduit (204a, 204b); and
the illumination light spread lens (236a, 236b) has an inner face operatively aligned with an illumination light output of the illumination light conduit (204a, 204b).

3. The distal head (106) of claim 2 wherein the operating light spectrum for the image sensor-based camera assembly (224) and the illumination light conduit (204a, 204b) is the visible light spectrum.

4. The distal head (106) of claim 2 or 3 wherein the illumination light spread lens (236a, b) is a plano-concave lens having a concave side (238a, 238 b) comprising the inner face of the illumination light spread lens (236a, 236b).

5. The distal head (106) according to one of the claims 1 to 4 wherein the illumination light conduit (204a, 204b) comprises a plastic optical fiber.

6. The distal (106) head according to one of the claims 1 to 5 wherein the first, second, and third light-cured securing material (240) comprises UV-cured epoxy.

7. An endoscope (100) including:
an elongated endoscope shaft (101) having a distal end (105) and a proximal end (104);
an endoscope handle (102) connected at the proximal end (104) of the elongated endoscope shaft (101);
a distal head housing (201) according to one of the claims 1 to 6 connected at the distal end (105) of the elongated endoscope shaft (101), the distal head housing (201) defining a distal end surface of the endoscope (100);
the working channel tube (210) extending from a working channel access structure of the endoscope handle (102) through the elongated endoscope shaft (101) and to a working channel tube end secured within the working channel tube receiving cavity with the first light-cured securing material (240) cured in response to light within the securing material curing spectrum;
the image sensor-based camera assembly (224) being connected to a power and signal conduit arrangement that extends from the image sensor-based camera assembly (224) through the elongated endoscope shaft (101) to the endoscope handle (102); and
the illumination light conduit (204a, 204b) having a distal end secured within the illumination light conduit receiving cavity with the third light-cured securing material (240) cured in response to light within the securing material curing spectrum, the illumination light conduit (204a, 204b) extending through the elongated endoscope shaft (101) and being operably coupled at a proximal end to an illumination light source associated with the endoscope handle (102).

8. The endoscope (100) of claim 7 wherein the illumination spread lens (236a, 236b) has an inner face operatively aligned with an illumination light output of the illumination light conduit (204a, b).

9. The endoscope (100) of claim 7 or 8 wherein the illumination light spread lens (236a, 236b) is a plano-concave lens having a concave side (238a, 238b) comprising the inner face of the illumination light spread lens (236a, 236b) or the illumination light conduit (204a, 204b) comprises a plastic optical fiber.

10. The endoscope (100) according to one of the claims 7 to 9 wherein the first, second, and third light-curable securing material (240) comprises a UV-cured epoxy.

11. A method of manufacturing a distal head (106) of one of the claims 1 to 10 for an endoscope (100), the method including:
securing the distal head housing (201) in an assembly position;
placing the working channel tube (210), the image sensor-based camera assembly (224) and the illumination light conduit (204a, 204b) as endoscope head components in an operating position within the respective receiving cavities with a respective light-curable securing material interposed between a respective surface (228, 230, 232a, 232b) of said cavities and an outer surface of said endoscope head components (204a, 204b, 210, 224), the respective light-curable securing material being curable when subjected to light within the securing material curing spectrum; and
while maintaining said endoscope head components (204a, 204b, 210, 224) in the operating position within said cavities with the respective light-curable securing material interposed between the respective surface (228, 230, 232a, 232b) of said cavities and the outer surface of said endoscope head components (204a, 204b, 210, 224), directing light within the securing material curing spectrum through the light-permeable material of the distal head housing (201) to the respective light-curable securing material interposed between the respective surface (228, 230, 232a, 232b) of said cavities and the outer surface of said endoscope head components (204a, 204b, 210) to cure the respective light-curable securing material so as to secure said endoscope head components (204a, 204b, 210, 224) in the operating position within said cavities.

12. The method of claim 11 wherein at least two or more of the endoscope head components (204a, 204b, 208, 210, 224) are secured simultaneously by curing all of the respective light-curable securing material in the operating position within the respective receiving cavities.

13. The method of claim 11 wherein after curing the respective light-curable securing material so as to secure a first endoscope head component of the endoscope head components (204a, 204b, 208, 210, 224) two more of the endoscope head components (204a, 204b, 208, 210, 224) are secured simultaneously by curing the respective light-curable securing material in the operating position within the respective receiving cavities.

## Patentansprüche

1. Distaler Kopf (106) für ein Endoskop (100), wobei der distale Kopf (106) Folgendes einschließt:
ein distales Kopfgehäuse (201), das eine distale Endoberfläche des distalen Kopfes (106) definiert und aus einem lichtdurchlässigen Material gebildet ist, das für Licht innerhalb eines Befestigungsmaterialaushärtungsspektrums durchlässig ist;
einen Arbeitskanalrohr aufnehmenden Hohlraum, der innerhalb des distalen Kopfgehäuses (201) definiert ist; einen Kamera aufnehmenden Hohlraum, der innerhalb des distalen Kopfgehäuses (201) definiert ist;
eine Beleuchtungslichtstreulinse (236a, 236b), die integral in dem distalen Kopfgehäuse (201) aus dem lichtdurchlässigen Material gebildet ist;
einen Beleuchtungslichtleitung aufnehmenden Hohlraum, der innerhalb des distalen Kopfgehäuses (201) definiert ist; **gekennzeichnet durch**
ein Arbeitskanalrohr (210), das innerhalb des Arbeitskanalrohr aufnehmenden Hohlraums mit einem ersten lichtgehärteten Befestigungsmaterial (240) befestigt ist, das als Reaktion auf Licht innerhalb des Befestigungsmaterialaushärtungsspektrums ausgehärtet wird;
eine bildsensorbasierte Kameraanordnung (224), die innerhalb des Kamera aufnehmenden Hohlraums mit einem zweiten lichtgehärteten Befestigungsmaterial (240) befestigt ist, das als Reaktion auf Licht innerhalb des Befestigungsmaterialaushärtungsspektrums ausgehärtet wird; und
eine Beleuchtungslichtleitung (204a, 204b), die innerhalb des Beleuchtungslichtleitung aufnehmenden Hohlraums mit einem dritten lichtgehärteten Befestigungsmaterial (240) befestigt ist, das als Reaktion auf Licht innerhalb des Befestigungsmaterialaushärtungsspektrums ausgehärtet wird.

2. Distaler Kopf (106) nach Anspruch 1, wobei:
das lichtdurchlässige Material auch für Licht innerhalb eines Spektrums durchlässig ist, das ein Betriebslichtspektrum für die bildsensorbasierte Kameraanordnung (224) und die Beleuchtungslichtleitung (204a, 204b) einschließt; und
die Beleuchtungslichtstreulinse (236a, 236b) eine Innenfläche aufweist, die operativ mit einer Beleuchtungslichtausgabe der Beleuchtungslichtleitung (204a, 204b) ausgerichtet ist.

3. Distaler Kopf (106) nach Anspruch 2, wobei das Betriebslichtspektrum für die bildsensorbasierte Kameraanordnung (224) und die Beleuchtungslichtleitung (204a, 204b) das sichtbare Lichtspektrum ist.

4. Distaler Kopf (106) nach Anspruch 2 oder 3, wobei die Beleuchtungslichtstreulinse (236a, b) eine plankonkave Linse mit einer konkaven Seite (238a, 238b) ist, die die Innenfläche der Beleuchtungslichtstreulinse (236a, 236b) umfasst.

5. Distaler Kopf (106) nach einem der Ansprüche 1 bis 4, wobei die Beleuchtungslichtleitung (204a, 204b) eine optische Kunststofffaser umfasst.

6. Distaler Kopf (106) nach einem der Ansprüche 1 bis 5, wobei das erste, zweite und dritte lichtgehärtete Befestigungsmaterial (240) UV-gehärtetes Epoxid umfasst.

7. Endoskop (100), das Folgendes einschließt:
einen länglichen Endoskopschaft (101) mit einem distalen Ende (105) und einem proximalen Ende (104);
einen Endoskopgriff (102), der an dem proximalen Ende (104) des länglichen Endoskopschafts (101) verbunden ist; ein distales Kopfgehäuse (201) nach einem der Ansprüche 1 bis 6, das an dem distalen Ende (105) des länglichen Endoskopschafts (101) verbunden ist, wobei das distale Kopfgehäuse (201) eine distale Endoberfläche des Endoskops (100) definiert;
wobei sich das Arbeitskanalrohr (210) von einer Arbeitskanalzugangsstruktur des Endoskopgriffs (102) durch den länglichen Endoskopschaft (101) und zu einem Arbeitskanalrohrende erstreckt, das innerhalb des Arbeitskanalrohr aufnehmenden Hohlraums befestigt ist, wobei das erste lichtgehärtete Befestigungsmaterial (240) als Reaktion auf Licht innerhalb des Befestigungsmaterialaushärtungsspektrums ausgehärtet wird;
wobei die bildsensorbasierte Kameraanordnung (224) mit einer Leistungs- und Signalleitungsanordnung verbunden ist, die sich von der bildsensorbasierten Kameraanordnung (224) durch den länglichen Endoskopschaft (101) zu dem Endoskopgriff (102) erstreckt; und
wobei die Beleuchtungslichtleitung (204a, 204b) ein distales Ende aufweist, das innerhalb des Beleuchtungslichtleitung aufnehmenden Hohlraums befestigt ist, wobei das dritte lichtgehärtete Befestigungsmaterial (240) als Reaktion auf Licht innerhalb des Befestigungsmaterialaushärtungsspektrums ausgehärtet ist, wobei sich die Beleuchtungslichtleitung (204a, 204b) durch den länglichen Endoskopschaft (101) erstreckt und an einem proximalen Ende mit einer Beleuchtungslichtquelle, die mit dem Endoskopgriff (102) assoziiert ist, operativ gekoppelt ist.

8. Endoskop (100) nach Anspruch 7, wobei die Beleuchtungsstreulinse (236a, 236b) eine Innenfläche aufweist, die operativ mit einer Beleuchtungslichtausgabe der Beleuchtungslichtleitung (204a, b) ausgerichtet ist.

9. Endoskop (100) nach Anspruch 7 oder 8, wobei die Beleuchtungslichtstreulinse (236a, 236b) eine plankonkave Linse mit einer konkaven Seite (238a, 238b) ist, die die Innenfläche der Beleuchtungslichtstreulinse (236a, 236b) umfasst, oder die Beleuchtungslichtleitung (204a, 204b) eine optische Kunststofffaser umfasst.

10. Endoskop (100) nach einem der Ansprüche 7 bis 9, wobei das erste, zweite und dritte lichtaushärtbare Befestigungsmaterial (240) ein UV-gehärtetes Epoxid umfasst.

11. Verfahren zum Herstellen eines distalen Kopfes (106) nach einem der Ansprüche 1 bis 10 für ein Endoskop (100), wobei das Verfahren Folgendes einschließt:
Befestigen des distalen Kopfgehäuses (201) in einer Montageposition;
Platzieren des Arbeitskanalrohrs (210), der bildsensorbasierten Kameraanordnung (224) und der Beleuchtungslichtleitung (204a, 204b) als Endoskopkopfkomponenten in einer Betriebsposition innerhalb der jeweiligen aufnehmenden Hohlräume mit einem jeweiligen lichtaushärtbaren Befestigungsmaterial, das zwischen einer jeweiligen Oberfläche (228, 230, 232a, 232b) der Hohlräume und einer Außenoberfläche der Endoskopkopfkomponenten (204a, 204b, 210, 224) angeordnet ist, wobei das jeweilige lichtaushärtbare Befestigungsmaterial aushärtbar ist, wenn es Licht innerhalb des Befestigungsmaterialaushärtungsspektrums ausgesetzt wird; und
während die Endoskopkopfkomponenten (204a, 204b, 210, 224) in der Betriebsposition innerhalb der Hohlräume gehalten werden, wobei das jeweilige lichtaushärtbare Befestigungsmaterial zwischen der jeweiligen Oberfläche (228, 230, 232a, 232b) der Hohlräume und der Außenoberfläche der Endoskopkopfkomponenten (204 230a, 204b, 210, 224) angeordnet ist, Leiten von Licht innerhalb des Befestigungsmaterialaushärtungsspektrums durch das lichtdurchlässige Material des distalen Kopfgehäuses (201) zu dem jeweiligen lichtaushärtbaren Befestigungsmaterial, das zwischen der jeweiligen Oberfläche (228 232a, 232b) der Hohlräume und der Außenoberfläche der Endoskopkopfkomponenten (204a, 204b, 210) angeordnet ist, um das jeweilige lichtaushärtbare Befestigungsmaterial auszuhärten, um die Endoskopkopfkomponenten (204a, 204b, 210, 224) in der Betriebsposition innerhalb der Hohlräume zu befestigen.

12. Verfahren nach Anspruch 11, wobei mindestens zwei oder mehr der Endoskopkopfkomponenten (204a, 204b, 208, 210, 224) gleichzeitig durch Aushärten des gesamten jeweiligen lichtaushärtbaren Befestigungsmaterials in der Betriebsposition innerhalb der jeweiligen aufnehmenden Hohlräume befestigt werden.

13. Verfahren nach Anspruch 11, wobei nach dem Aushärten des jeweiligen lichtaushärtbaren Befestigungsmaterials, um eine erste Endoskopkopfkomponente der Endoskopkopfkomponenten (204a, 204b, 208, 210, 224) zu befestigen, zwei weitere der Endoskopkopfkomponenten (204a, 204b, 208, 210, 224) gleichzeitig durch Aushärten des jeweiligen lichtaushärtbaren Befestigungsmaterials in der Betriebsposition innerhalb der jeweiligen aufnehmenden Hohlräume befestigt werden.

## Revendications

1. Tête distale (106) pour un endoscope (100), la tête distale (106) comportant :
une enveloppe de tête distale (201) définissant une surface d'extrémité distale de la tête distale (106) et étant formée à partir d'un matériau perméable à la lumière qui est perméable à la lumière à l'intérieur d'un spectre de durcissement de matériau de fixation ;
une cavité de réception de tube à canal de travail définie à l'intérieur de l'enveloppe de tête distale (201) ;
une cavité de réception de caméra définie à l'intérieur de l'enveloppe de tête distale (201) ;
une lentille d'étalement de lumière d'éclairage (236a, 236b) formée d'un seul tenant dans l'enveloppe de tête distale (201) à partir du matériau perméable à la lumière ;
une cavité de réception de conduit de lumière d'éclairage définie à l'intérieur de l'enveloppe de tête distale (201) ; **caractérisé par**
un tube à canal de travail (210) fixé à l'intérieur de la cavité de réception de tube à canal de travail avec un premier matériau de fixation photodurci (240) durci en réponse à la lumière à l'intérieur du spectre de durcissement de matériau de fixation ;
un ensemble caméra à base de capteurs d'images (224) fixé à l'intérieur de la cavité de réception de caméra avec un deuxième matériau de fixation photodurci (240) durci en réponse à la lumière à l'intérieur du spectre de durcissement de matériau de fixation ; et
un conduit de lumière d'éclairage (204a, 204b) fixé à l'intérieur de la cavité de réception de conduit de lumière d'éclairage avec un troisième matériau de fixation photodurci (240) durci en réponse à la lumière à l'intérieur du spectre de durcissement de matériau de fixation.

2. Tête distale (106) selon la revendication 1 dans laquelle :
le matériau perméable à la lumière est également perméable à la lumière à l'intérieur d'un spectre qui inclut un spectre de lumière de fonctionnement pour l'ensemble caméra à base de capteurs d'images (224) et le conduit de lumière d'éclairage (204a, 204b) ; et
la lentille d'étalement de lumière d'éclairage (236a, 236b) a une face interne fonctionnellement alignée avec une sortie de lumière d'éclairage du conduit de lumière d'éclairage (204a, 204b).

3. Tête distale (106) selon la revendication 2 dans laquelle le spectre de lumière de fonctionnement pour l'ensemble caméra à base de capteurs d'images (224) et le conduit de lumière d'éclairage (204a, 204b) est le spectre de lumière visible.

4. Tête distale (106) selon la revendication 2 ou 3 dans laquelle la lentille d'étalement de lumière d'éclairage (236a, b) est une lentille plan-concave ayant un côté concave (238a, 238b) constituant la face interne de la lentille d'étalement de lumière d'éclairage (236a, 236b).

5. Tête distale (106) selon une des revendications 1 à 4 dans laquelle le conduit de lumière d'éclairage (204a, 204b) comprend une fibre optique en plastique.

6. Tête distale (106) selon une des revendications 1 à 5 dans laquelle les premier, deuxième et troisième matériaux de fixation photodurcis (240) comprennent de la résine époxy durcie aux UV.

7. Endoscope (100) comportant :
une tige d'endoscope allongée (101) ayant une extrémité distale (105) et une extrémité proximale (104) ;
une poignée d'endoscope (102) reliée à l'extrémité proximale (104) de la tige d'endoscope allongée (101) ;
une enveloppe de tête distale (201) selon une des revendications 1 à 6 reliée à l'extrémité distale (105) de la tige d'endoscope allongée (101), l'enveloppe de tête distale (201) définissant une surface d'extrémité distale de l'endoscope (100) ;
le tube à canal de travail (210) s'étendant depuis une structure d'accès au canal de travail de la poignée d'endoscope (102) à travers la tige d'endoscope allongée (101) et jusqu'à une extrémité de tube à canal de travail fixée à l'intérieur de la cavité de réception de tube à canal de travail avec le premier matériau de fixation photodurci (240) durci en réponse à la lumière à l'intérieur du spectre de durcissement de matériau de fixation ;
l'ensemble caméra à base de capteurs d'images (224) étant relié à un agencement de conduits d'alimentation et de signaux qui s'étend depuis l'ensemble caméra à base de capteurs d'images (224) à travers la tige d'endoscope allongée (101) jusqu'à la poignée d'endoscope (102) ; et
le conduit de lumière d'éclairage (204a, 204b) ayant une extrémité distale fixée à l'intérieur de la cavité de réception de conduit de lumière d'éclairage avec le troisième matériau de fixation photodurci (240) durci en réponse à la lumière à l'intérieur du spectre de durcissement de matériau de fixation, le conduit de lumière d'éclairage (204a, 204b) s'étendant à travers la tige d'endoscope allongée (101) et étant fonctionnellement couplé à une extrémité proximale à une source de lumière d'éclairage associée à la poignée d'endoscope (102).

8. Endoscope (100) selon la revendication 7 dans lequel la lentille d'étalement d'éclairage (236a, 236b) a une face interne fonctionnellement alignée avec une sortie de lumière d'éclairage du conduit de lumière d'éclairage (204a, b).

9. Endoscope (100) selon la revendication 7 ou 8 dans lequel la lentille d'étalement de lumière d'éclairage (236a, 236b) est une lentille plan-concave ayant un côté concave (238a, 238b) constituant la face interne de la lentille d'étalement de lumière d'éclairage (236a, 236b) ou bien le conduit de lumière d'éclairage (204a, 204b) comprend une fibre optique en plastique.

10. Endoscope (100) selon une des revendications 7 à 9 dans lequel les premier, deuxième et troisième matériaux de fixation photodurcis (240) comprennent de la résine époxy durcie aux UV.

11. Procédé de fabrication d'une tête distale (106) d'une des revendications 1 à 10 pour un endoscope (100), le procédé comportant :
la fixation de l'enveloppe de tête distale (201) dans une position d'assemblage ;
la mise en place du tube à canal de travail (210), de l'ensemble caméra à base de capteurs d'images (224) et du conduit de lumière d'éclairage (204a, 204b) en tant que composants de tête d'endoscope dans une position de fonctionnement à l'intérieur des cavités de réception respectives avec un matériau de fixation photodurcissable respectif intercalé entre une surface respective (228, 230, 232a, 232b) desdites cavités et une surface externe desdits composants de tête d'endoscope (204a, 204b, 210, 224), le matériau de fixation photodurcissable respectif étant durcissable quand il est soumis à la lumière à l'intérieur du spectre de durcissement de matériau de fixation ; et
tout en maintenant lesdits composants de tête d'endoscope (204a, 204b, 210, 224) dans la position de fonctionnement à l'intérieur desdites cavités avec le matériau de fixation photodurcissable respectif intercalé entre la surface respective (228, 230, 232a, 232b) desdites cavités et la surface externe desdits composants de tête d'endoscope (204a, 204b, 210, 224), l'acheminement de lumière à l'intérieur du spectre de durcissement de matériau de fixation à travers le matériau perméable à la lumière de l'enveloppe de tête distale (201) jusqu'au matériau de fixation photodurcissable respectif intercalé entre la surface respective (228, 230, 232a, 232b) desdites cavités et la surface externe desdits composants de tête d'endoscope (204a, 204b, 210) pour durcir le matériau de fixation photodurcissable respectif de manière à fixer lesdits composants de tête d'endoscope (204a, 204b, 210, 224) dans la position de fonctionnement à l'intérieur desdites cavités.

12. Procédé selon la revendication 11 dans lequel au moins deux des composants de tête d'endoscope (204a, 204b, 208, 210, 224) ou plus sont fixés simultanément par durcissement de tous les matériaux de fixation photodurcissables respectifs dans la position de fonctionnement à l'intérieur des cavités de réception respectives.

13. Procédé selon la revendication 11 dans lequel, après le durcissement du matériau de fixation photodurcissable respectif de manière à fixer un premier composant de tête d'endoscope parmi les composants de tête d'endoscope (204a, 204b, 208, 210, 224), deux autres composants de tête d'endoscope (204a, 204b, 208, 210, 224) sont fixés simultanément par durcissement du matériau de fixation photodurcissable respectif dans la position de fonctionnement à l'intérieur des cavités de réception respectives.
